(19) Europäisches Patentamt
European Patent Office
Office européen des brevets

(11) **EP 4 796 023 A1**

(12) **EUROPEAN PATENT APPLICATION**

(43) Date of publication:
**26.08.2026 Bulletin 2026/35**

(21) Application number: **25305231.0**

(22) Date of filing: **20.02.2025**

(51) International Patent Classification (IPC):
***A61B 5/00*** *(2006.01)* ***G06N 3/049*** *(2023.01)*

(52) Cooperative Patent Classification (CPC):
**A61B 5/7275; G06N 3/049**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Dassault Systèmes**
**78140 Vélizy-Villacoublay (FR)**

(72) Inventors:
- **GUYONVARCH, Romain**
**78140 Vélizy-Villacoublay (FR)**
- **MARGATE, Tristan**
**78140 Vélizy-Villacoublay (FR)**
- **ZULIAN, Marine**
**06560 Valbonne (FR)**

(74) Representative: **Bandpay & Greuter**
**11, rue Christophe Colomb**
**75008 Paris (FR)**

# (54) ENCODING A TIME SERIES OF VALUES OF A PHYSIOLOGICAL MEASURE OF A PATIENT

(57) The disclosure notably relates to a computer-implemented method for encoding a time series of values of a physiological measure of a patient. The method comprises obtaining the time series of values of the physiological measure of the patient. The method further comprises, for each time point of the time series of values, obtaining a slope coefficient of the time series of values at the time point. The obtaining a slope coefficient is based on the time point, a predetermined number of previous time points, and a predetermined number of next time points. The method further comprises, for each time point of the time series of values, comparing the slope coefficient to a reference slope coefficient. The method is an improved solution for encoding a time series of values of a physiological measure of a patient.

EP 4 796 023 A1

## Description

### TECHNICAL FIELD

The disclosure relates to the field of computer programs and systems, and more specifically to a method, system and program for encoding a time series of values of a physiological measure of a patient.

### BACKGROUND

In the context of predicting the evolution of a disease of a patient, it is beneficial to employ mathematical models able to estimate the probability of clinical events occurring over time. These events can include disease relapse, progression to more severe stages, complications, hospitalizations, or patient mortality. The primary objective of utilizing such models is to predict as accurately as possible the occurrence of clinical events, such as death for example.

Mathematical models used for these predictions encompass a range of approaches, including statistical models and machine learning algorithms. The input data for these models typically consist of longitudinal patient information collected over multiple time points during successive visits to healthcare specialists. This data may include laboratory results, imaging findings, and vital signs. By leveraging such data, the models can identify patterns and trends that may not be immediately apparent, thereby enhancing the accuracy of predictions. These models may thus perform indirect measurements of medical/physiological features based on measured physiological data of the patient.

Some of the models used for such predictive solutions may only be able to process inputs under the form of series of spikes. Prior art methods used for encoding input data to series of spikes may fail to properly encode the input data, overly representing the noise present in the input data.

Within this context, there is still a need for an improved solution for encoding a time series of values of a physiological measure of a patient.

### SUMMARY

It is therefore provided a computer-implemented method for encoding a time series of values of a physiological measure of a patient. The method may be referred to as "the encoding method" in the present disclosure.

The encoding method comprises obtaining the time series of values of the physiological measure of the patient. The encoding method further comprises, for each time point of the time series of values, obtaining a slope coefficient of the time series of values at the time point. The obtaining a slope coefficient is based on the time point, a predetermined number of previous time points, and a predetermined number of next time points. The encoding method further comprises, for each time point of the time series of values, comparing the slope coefficient to a reference slope coefficient. The comparing the slope coefficient comprises, if the computed slope coefficient is above the reference slope coefficient in absolute value, encoding the time point as a spike. The comparing the slope coefficient further comprises, if the computed slope is below the reference slope in absolute value, encoding the time point as no spike.

The encoding method may comprise one or more of the following:

- the obtaining a slope coefficient of the time series of values at the time point comprises computing a linear regression on the values of the time series comprising the time point, the predetermined number of previous time points, and the predetermined number of next time points;
- the reference slope is 1 or a quotient of a maximum amplitude of the values of the time series and a maximum time amplitude;
- each of the spikes is either a positive spike or a negative spike, and comparing the slope coefficient to a reference coefficient further comprises:
    - o if the computed slope coefficient is above the reference slope coefficient in absolute value, encoding the time point as a positive spike; and
    - o if the computed slope coefficient is below the opposite of the reference slope coefficient in absolute value, encoding the time point as a negative spike; and/or
- providing the encoded time series of values of the physiological measure of the patient to a neural network comprising a Spike Neural Network (SNN) for predicting a time occurrence of a clinical/medical event.

It is further provided a computer program comprising instructions for performing the encoding method.

It is further provided a computer readable storage medium having recorded thereon the computer program.

It is further provided a system comprising a processor coupled to a memory and a graphical user interface, the

memory having recorded thereon the computer program.

## BRIEF DESCRIPTION OF THE DRAWINGS

Non-limiting examples will now be described in reference to the accompanying drawings, where:

- FIGs. 1-10 illustrate examples of implementations of the method; and
- FIG. 11 shows an example of the system.

## DETAILED DESCRIPTION

It is proposed a computer-implemented method for machine-learning a neural network comprising a spike neural network (SNN in short) for predicting a time occurrence of a clinical event. The method may be referred to as "the learning method" in the present disclosure.

The learning method comprises obtaining a training dataset of training examples. Each training example comprises one or more time series of values (e.g., of measurements) of respective physiological measures (i.e., each time series of values being a time series of a respective physiological measure) of a patient (a same patient for each of the one or more time series of values) over a past time period and a corresponding ground truth of a time occurrence of the clinical event. The learning method further comprises training the neural network based on the training dataset. The neural network is trained for taking as input one or more time series of values of the respective physiological measures of a patient over a past time period and for outputting a prediction of probabilities over a time period of the occurrence of the clinical event.

In the present disclosure, a physiological measure is any characteristic related to a patient that can be obtained and/or measured, e.g., by a health specialist, e.g., with a medical measuring device. A physiological measure is a measure that change over time such as, as non-limiting examples, blood pressure, heart rate, body temperature, results of blood tests, urine tests, radiographies, or a combination thereof. The physiological measures are also referred to as longitudinal measures. Physiological measures may be obtained, e.g., with medical measuring devices and/or in laboratories.

In the present disclosure, a clinical event is any occurrence that is observable and/or measurable and that is significant to a patient's health. A clinical event is associated with a time of occurrence, which is a time point (e.g., an hour, a day, or a month) in which the clinical event first manifested. A clinical event may be any measurable or observable change in a patient's overall health status, including the patient's death. Additionally or alternatively, a clinical event may be associated with a specific disease and may include, but is not limited to, the onset of a disease, its relapse, or a subsequent recovery.

In the present disclosure, a prediction of probabilities over a time period of an occurrence of a clinical event may be a sequence of probabilities over a period (e.g., a probability for each day, or for each month, of the time period, for example probability for each day over 300 days or the coming year) indicating the likelihood that the clinical event occurs at each of the specified time point of the time period. The time points may be distributed regularly in the time period (e.g., every day/month of the time period). The probability may indicate a confidence level of the method in the prediction of the occurrence of a clinical event. The time period may, e.g., coincide with the time period of the training data or may be a time period in the future (e.g., the time period may be a span of time starting right after the method is used). Therefore, the methods of the present disclosure may be both used to predict a clinical event in the future, or to obtain information on a first occurrence of a clinical event that has already occurred (e.g., a first occurrence in the past of a cancer relapse). The span of time may be days, months, or years, e.g., 300 days or 3 years.

In implementations of the methods of the present disclosure, the clinical event may be associated with a cancer and an example of a clinical event may be the relapse of the cancer, characterized by the reappearance or progression of malignant cells (e.g., following a period of remission). A prediction of a time occurrence of relapse, may be an anticipated moment when the cancer is expected to relapse, and the associated probability may be the confidence level of the method in the prediction.

In the present disclosure, a time series of values of a (respective) physiological measure consists of one or more values (e.g., measurements) of the physiological measure of a patient, each of the values being associated to a time point (also called time step). Each time point may represent a specific moment in time (e.g., an hour, a day, or a month) that may indicate the moment in time at which the value was obtained (e.g., when a measurement was made). In examples, each of the one or more time series of values of respective physiological measures of a patient may comprise one or more time series, each time series comprising entries, each entry comprising a value and/or measurement of a respective physiological measure of the patient and a date (e.g., an hour, a day, or a month) at which the measurement was performed. In examples, each time series may be expressed as a vector, the index of the vector corresponding to the time point, and the value at each index corresponding to a value and/or measurement of the respective physiological measure

of the patient at the respective time point. In formulae, each time series may be expressed as a sequence $(X_t)_{t=0}^{n}$, the index $t$ expressing a time point (e.g., each index being associated to a different hour, day, and/or month) and the value $X_t$ expressing the value of the respective physiological measure at the time point t.

The methods of the present disclosure may comprise obtaining one or more time series of values of respective physiological measures of a patient by collecting the measurements of the physiological measures of the patient at different time points, e.g., one or more values at each visit of the patient to a health specialist. Therefore, one or more of the values of the physiological measures may be obtained, e.g., by a health specialist, e.g., by measuring with a medical measuring device. For example, a patient might regularly visit a health specialist and undergo a blood test/urine test during each appointment. Therefore, each time series of a respective physiological measure of a patient may be composed of values of the respective physiological measure obtained at irregular intervals, e.g., corresponding to visits of the patient to a health specialist. The irregularity of these intervals poses a major challenge to training neural networks and deep learning algorithms. Indeed, many of the models used in prior art methods do not, and cannot, take into account these irregularities.

The methods of the present disclosure may comprise normalizing the values of the obtained time series of values of respective physiological measures of a patient. In formulae, the values $X_t$ may be normalized, with any method known in the art (e.g., considering a mean and/or a standard deviation).

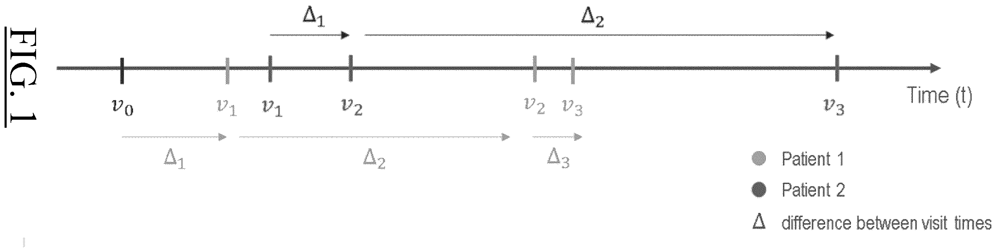

FIG. 1 illustrates an example of irregular intervals visits of two patients.

Moreover, the training dataset comprising one or more time series of values of respective physiological measures of a patient over a past time period and corresponding ground truth of a time occurrence of the clinical event may be incomplete. This may be due to the fact that the data of a patient may not be available after a period of time, e.g., due to the transfer of the patient to another hospital (e.g., another city) or an end of a clinical study/trial. This creates an additional difficulty in training deep learning models based on such data.

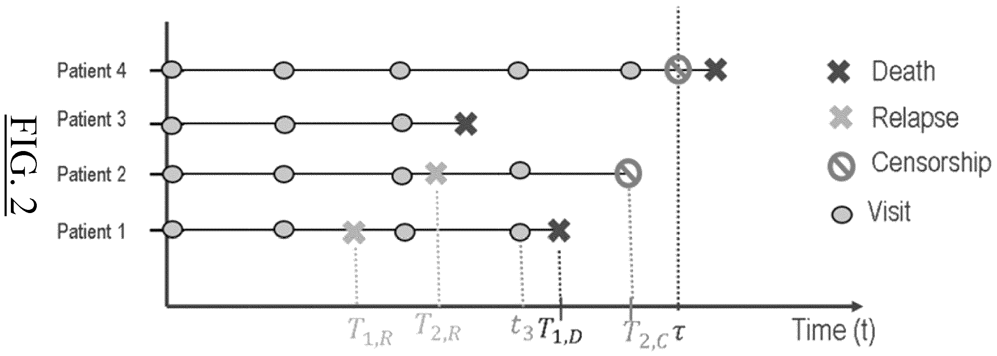

FIG. 2 illustrates an example of the history of visits and clinical events of four different patients.

The analysis of time series of values of respective physiological measures (e.g., the analysis of the evolution of longitudinal characteristics) can be important for understanding progression of diseases, for evaluating responses to medical treatments, and for assessing the overall impact of pathologies on patients. For example, some physiological measures, or their change/evolution over time, may correlate with a specific disease. In a concrete example, the evolution of the presence and/or concentration of some specific proteins may be of importance for understanding the evolution of a cancer. In a specific example, the Prostate Specific Antigen (PSA), and its evolution over time, can be of importance for prostate cancer, as it is shown that it may be an indicator of the relapse of such a cancer.

However, in most cases, the correlation between time series of values of respective physiological measures and clinical events (e.g., related to a disease) may be complex. In examples, the combination of the evolution of several different physiological measures, the combination comprising different weights and complex mathematical functions, may be a best predictor of a given clinical event (e.g., related to a disease). As such combinations may be very complex and depend on the specific disease, it may not be feasible to have a (e.g., different, or specific) mathematical model for each of the clinical events related to each of the diseases. Health specialists often rely on their (biased) personal experience for these predictions, by mentally comparing the specific situation of the patient with other cases known by the health specialist. Such predictions are often imprecise and may strongly depend on the health specialist.

The learning method is an improved solution for learning a neural network for predicting a time occurrence of a clinical event.

Indeed, the learning method provides a method that may be used for any possible clinical event, such as clinical events associated with diseases. The neural network trained by the learning method may automatically learn to predict the occurrence of a clinical event, from a training dataset. As a non-limiting example, the dataset may be a clinical study for the given clinical event and/or disease. Thereby, the learning method can train a neural network able to output a prediction of probabilities over a time period of the occurrence of the clinical event, starting from data obtained from a clinical study. Therefore, the learning method removes the necessity of having a specific mathematical model for each of the different clinical events, offering a unified framework.

In implementations of the learning method, the clinical event may be related to a cancer, e.g., the relapse of a cancer, or the death of a patient due to the cancer. Each physiological measure may be a measure of particular relevance for cancer. As a non-limiting example, the physiological measure may be the concentration of a specific molecule, such as a protein, in the body (e.g., as detected in a blood test, or in a urine test). Alternatively, a physiological measure may be a size (e.g., a diagonal, a volume) of a tumor, as measured in radiography. The data may be acquired from a clinical study for cancer. The learning method thereby, can machine learn/train a neural network to predict the relapse of the cancer and/or the death of a patient due to cancer, starting from the above-mentioned measurements.

A neural network machine-learnt/trained using the learning method may be used by a health specialist. The health specialist may give as input to the trained neural network the longitudinal data accumulated over time about a patient (i.e., one or more time series of values of respective physiological measures of a patient). Using the neural network, the health specialist may, therefore, measure and/or predict a (e.g., future) occurrence of the clinical event (such as death

or a patient due to cancer), with an accuracy that would not be possible when, for example, making a guess based on the professional's experience. Such measure may be used to obtain an indirect measure of the efficacy of a treatment, or of the progression of a disease. Moreover, as will be clear in the implementation below, the method may be used to obtain an indirect measure of the size of a tumor.

In an implementation, the learning method was tested on a dataset comprising time series of values of respective physiological measures of patients with metastatic prostate cancer. Metastatic prostate cancer is an advanced stage of the disease. In this situation, the tumor has spread to other parts of the body beyond the tissue surrounding the prostate. The five-year survival chance of a patient with metastatic prostate cancer is around 50%. The data used in the implementation comes from four different studies of patients with metastatic prostate cancer. The data contains both longitudinal variables (such as PSA) and general patient information variables (such as BMI, and medical history). Prostate-specific antigen (PSA) is a protein produced by prostate cells. Variations in the concentration in the blood of the PSA is an important indicator of the evolution of tumor size. The dataset used contained data about more than 1000 prostate cancer patients. For each patient, the data comprises longitudinal data obtained during several visits to a health specialist, discretized in days, the first time corresponding to the start of the clinical study. The clinical event studied is the patient's death over time. The implementation was able to correctly predict the probability of the patient's death over time.

Moreover, contrary to the prior art algorithms that did not take into account the irregularity between patient visits times in the prediction task, the neural network of the learning method, by construction, takes into account the temporal irregularity in each time series of values of a respective physiological measure. This is achieved thanks to the spiking neural network. As it will be described in detail below in the disclosure, a spiking neural network has a temporal dimension and is capable of properly processing data obtained at irregular time intervals.

Therefore, the neural network learnt by the learning method, is able to more precisely predict a clinical event, compared to prior art methods.

It is further proposed a computer-implemented method of use of a neural network learnt according to the learning method. The method may be referred to as "the method of use" in the present disclosure.

The method of use comprises obtaining one or more time series values of respective physiological measures of a patient over a past time period. The method of use further comprises applying the neural network to the one or more time series, thereby predicting probabilities over a time period of the occurrence of the clinical event.

In implementations, the learning method and the method of use may be combined together. In implementations, either of the methods may comprise steps of the other method (e.g., the entire other method).

For the reasons given above, the method of use constitutes an improved solution to predicting a clinical event of a patient. The method of use may be used to accurately predict the timing of the clinical event.

In implementations, the clinical event may be the relapse of a disease, such as cancer. The relapse of cancer may be characterized and measured by the reappearance or progression of malignant cells, e.g., following a period of remission. Thereby the method of use may be used to measure the progression of malignant cells. In other implementations, the clinical event may be the death of a patient.

Such implementations may be used, for example, by a health specialist. Thereby, the health specialist may be able to measure the efficacy of a treatment, by providing a predictive analysis of its impact on cancer relapse and/or patient's death.

In the present disclosure, and as is known *per se* from machine-learning, a neural network may be defined by its architecture, parameters, and hyperparameters. The architecture consists of layers, starting with the input layer whose neuron count is equal to the dimensionality of the input data. The input layer may be followed by several hidden layers with a given number of neurons and activation functions. These layers and neurons define the network's depth and width. A neural network may comprise activation functions, e.g., in between layers, which may introduce non-linearity into the model. The output layer may have as many neurons as the variables in the output data. In implementations, the output layer may comprise a neuron for each of the considered time points of the time period. The interconnections between these layers define the topology of the neural network. The parameters of the neural network are the learnable weights and biases, which are determined/configured/modified in the training process. In contrast, the hyperparameters are predefined values/settings that are not learned from the training data. These encompasses the number of hidden layers, neurons per layer and much more.

The main difference between spiking neural networks (SNN) and traditional neural networks is the different structure of each artificial neuron cell. Spiking neural networks are made of spiking neurons. Spiking neurons model more closely the biological neuron cell and can handle temporal data. The learning method and the method of use leverage this ability to handle temporal data to take into account the irregularities of each time series of values of a respective physiological measure of a patient. This ability to handle temporal data makes the learnt neural network (according to the learning method) more accurate in the prediction, compared to previous art models. Spiking neurons take as input and give as output signals, each signal comprising a series of spikes. Spiking neurons use a membrane potential system, heavily inspired by mathematical model of biological neurons. Spiking neurons comprise a threshold, used to decide when and/or whether they should emit a spike.

In the present disclosure, a spiking neural network (SNN) may be defined by its architecture, parameters, and hyperparameters. The architecture is composed of (hidden) layers of spiking neurons that communicate via discrete events (spikes) rather than continuous activations. The number of (hidden) layers and the number of neurons per layer may vary depending on each implementation and may be determined after several benchmarks (e.g., depending on the training dataset and/or on the clinical event). The first layer of a spiking neural network may be an input layer where each neuron may correspond to a dimension or feature of the input data. Following the input layer, one or more hidden layers of spiking neurons process the incoming signals.

In the present disclosure, each spiking neural network, and each spiking neuron composing the spiking neural network, has a temporal dimension. The temporal dimension may be discretized and may depend on several time points. The difference between successive time points (herein, the time intervals) may be constant, so that the time points may be evenly distributed over a period of time. At each time point, each of the spiking neurons may receive as input and/or give as output a spike from/to one or more connected spiking neurons. The discretization, that is, the length of the time intervals, may depend on the specific implementations.

In implementations, each of the methods of the present disclosure may comprise normalizing in the time direction each of the obtained time series of values of respective physiological measures of a patient over a past time period before inputting each of the time series to the SNN. By normalizing, it is meant that the time intervals, that is, the difference between consecutive time points, may all be multiples of a basic time interval. The normalizing may consist in approximating each of the time point to the closest time point consisting of a multiple of the basic time interval. The basic time interval may be one or more days, or one or more weeks, e.g., one day or one month. In such implementation, the time series may be provided as input to the neural network learnt by the learning method and/or of the method of use, and the basic time interval may be the same as the time interval used in the SNN, as described above. Alternatively, in other implementations, the neural network may comprise normalizing in the time direction each of the time series of values of respective physiological measures of a patient over a past time period provided as input.

In the present disclosure, each given spiking neuron may have an internal memory (e.g., a membrane potential) that may depend on a time point. The memory may be expressed by a real number and may comprise the information of the number of past spikes received by the spiking neuron. The memory of past spikes may decrease over time, so that spikes received further in the past may contribute less to the memory.

In implementations of the methods of the present disclosure, each given spiking neuron may have a membrane potential $U_t$ depending on a time variable $t$ and may output a value $S_t \in \{0,1\}$ at each time t (e.g., fire a spike if $S_t = 1$ and not fire a spike if $S_t = 0$). The membrane potential $U_t$ and the output $S_t$ may be updated at each time step at follows:

$$U_{t+1} = I_{t+1} + \beta U_t - S_t U_{th}$$

$$S_{t+1} = \mathbb{1}_{U_{t+1} \geq U_{th}}$$

where:

- $I_{t+1}$ is the sum of all received spikes (or, equivalently, the sum of all the quantities $S_{t+1}$ of respective spiking neurons) at the time point t + 1, where the sum ranges over all spiking neurons (forward) connected to the given spiking neuron;
- $\beta \in [0,1]$ is a decay factor; and
- $U_{th}$ is a threshold value.

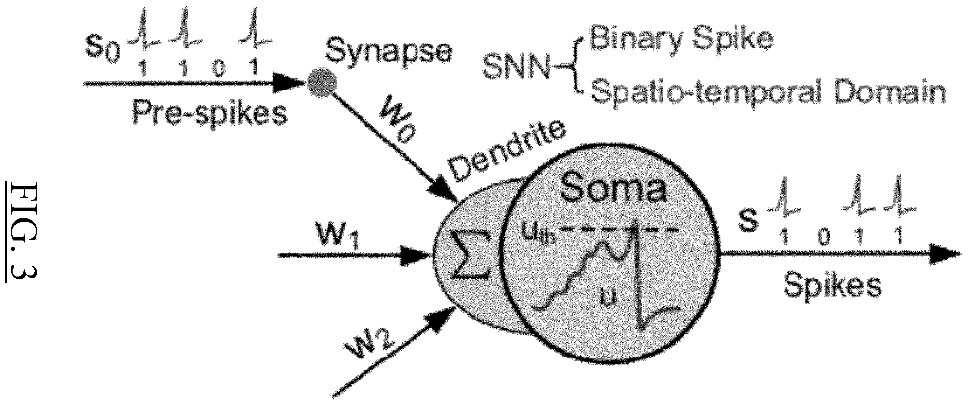

FIG. 3 shows an illustration of a spiking neuron.

In implementations of the methods of the present disclosure, each spike may contribute to a value of 1. In other words, each spike may be encoded as an emission of a value of 1. For example, the above defined function $S_t$ may be 1 if at time t the spiking neuron outputs a spike, and $S_t$ may be 0 otherwise.

In other implementations of the methods of the present disclosure, each spike may be a positive spike or a negative spike. A positive spike may contribute to a value of 1, whereas a negative spike may contribute to a value of -1. In other words, each positive spike may be encoded as an emission of a value of 1, and each negative spike may be encoded as an emission of a value of -1. In formulae, the above defined function $S_t$ may be 1 if at time t the spiking neuron outputs a positive spike, $S_t$ may be -1 if at time t the spiking neuron outputs a negative spike, and $S_t$ may be 0 otherwise. In such implementations, each given spiking neuron may comprise a positive threshold $U_{th}^{+}$ and a negative threshold $U_{th}^{-}$ such that whenever the membrane potential (e.g., memory) $U_t$ is higher than the positive threshold, then a positive spike is outputted/fired, and whenever the membrane potential (e.g., memory) $U_t$ is lower than the negative threshold, then a negative spike is outputted/fired. In implementations, the negative threshold may be the opposite of the positive threshold,

$$U_{th}^{-} = -U_{th}^{+} .$$

In yet other implementations of the methods of the present disclosure, each spike may be associated with a number (e.g., an integer number, or any other subset of the real numbers), may contribute to a value equal to the associated number to the above sum $I_t$, and the spiking neural network may comprise a respective threshold for each different number that may be associated with a spike.

In the present disclosure, therefore, spiking neurons may take as input and output signals. Signals may be sequences indexed by the time points t, so that their length may be the number of time points, and at each time point recording whether the associated spiking neuron has outputted a spike (and if yes, eventually of which kind) or not.

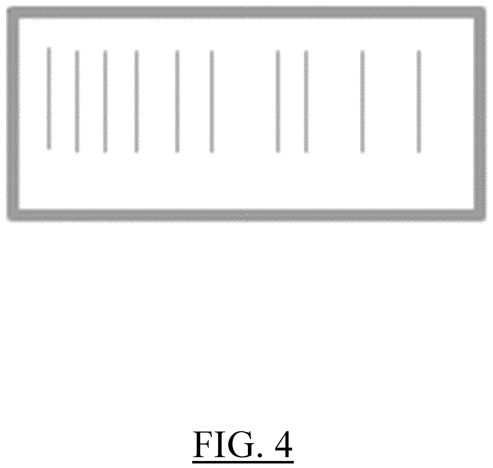

FIG. 4 illustrates a signal, comprising several spikes.

In implementations, signals may be represented by sequences of binary values, e.g., each value of the sequence being either "0" or "1". In such implementations, the value 0 may correspond to the absence of a spike, and 1 the presence of a spike. In other implementations, signals may be represented by sequences of values, each value being one of a list, the list comprising "0", "-1", and "1". The values may correspond to the absence of spikes, negative spikes, and positive spikes respectively. In yet other implementations, signals may be sequences of numbers, identifying associated spikes.

The learning method comprises obtaining a training dataset of training examples. The training dataset is to be used to machine-learning/training the neural network as it will be explained below in the present disclosure. Each training example comprises one or more time series of values of respective physiological measures of a patient over a past time period. Each training example is associated with a patient and may be associated with one or more physiological measures, comprising a time series of values of each of the physiological measures of the patient. The time series of values may be, as explained above, obtained by measuring the physiological measures of the patient repeatedly over time, the time series also comprising the time point at which each of the values is obtained. The values are obtained over the past time period, that is, the time points are in the past time period. The values may have been obtained by a health specialist, e.g., by measuring with a medical measuring device. Any training dataset herein may comprise a number of training examples, e.g., higher than 10000, or 50000.

Each training example further comprises a corresponding ground truth of a time occurrence of the clinical event. That is, each training example comprises the information of whether the clinical event has occurred for the patient associated with the training example (e.g., whether there was cancer relapse, or whether the patient died). If said clinical event has occurred, the training example comprises a time of occurrence of the clinical event. The ground truth is important for training the neural network, as during training the result of the providing the one or more time series to the neural network is compared to the ground truth.

In implementations, obtaining a training dataset of training examples may comprise data preparation and/or data preprocessing. Indeed, different clinical studies may concern different physiological measures and large amount of data may be missing.

In implementations, a past time period may be a time window (e.g., the duration of a clinical trial/study) and may be fixed. A time window may comprise two dates, a starting date and an end date, the two dates separated by a span of time. The span of time may be one or more years, e.g., greater than 1 year, or 5 years.

The obtaining of a training dataset of training examples may comprise retrieving at least part of the training dataset from (e.g., local or distant) memory or receiving (e.g., from a remote system) at least part of the training dataset having been thereby acquired. The obtaining may comprise obtaining at least part of the training dataset by measuring a physiological measure of a patient and recording the occurrence of the clinical event for the patient. The obtaining may comprise obtaining at least part of the training dataset from a clinical trial/study.

The training dataset obtained in the learning method comprises training examples, each comprising one or more time series of values of respective physiological measures of a patient. Each of the physiological measures may be related to the clinical event (e.g., each of the physiological measures may be pertinent for the clinical event to predict). That is, the values (e.g., the measurements) of the physiological measures may be correlated with the clinical event, e.g., they may not be independent. Using only physiological measures related to the clinical event may be important, as the neural network machine-learnt/trained using the training dataset may learn said correlation. Therefore, the prediction made by the machine-learnt neural network may be based on said correlation. Indeed, using unrelated measures may bias the neural network in unwanted ways, by learning correlations that are purely present by chance.

In implementations, the training dataset obtained in the learning method may comprise, for each training example, an id, the id corresponding to a patient. This may allow the neural network to correlate different values of physiological measures of a same patient to predict a same clinical event. Each training example of the learning method may further comprise baseline data of the patient. Baseline data may comprise any patient-dependent data that may be used to predict the clinical event and that either remain relatively constant over time, or changes over time independently of a specific patient (e.g., the age of a patient). In examples, a baseline data may change in the same way for each patient (e.g., linearly for each patient, such as the age of a patient). Baseline data may comprise, as non-limiting examples, age, sex, BMI, height, genetic profile, medical history, and/or other (Boolean) values related to the state of the patient prior to a

clinical study. Baseline data depends only on the patient, and not on the specific time point. Baseline data may allow the neural network to better predict the clinical event.

The method was implemented, and a training dataset was obtained. The training dataset can be visualized in a tabular form and comprised the table below.

| id | label | sex | hepatome | edema | serBilir | albumin | alkaline | SGOT | platelets |
|---|---|---|---|---|---|---|---|---|---|
| 1 | 1 | 0 | 1 | 2 | 14.5 | 2.6 | 1718 | 138 | 190 |
| 1 | 1 | 0 | 1 | 2 | 21.3 | 2.94 | 1612 | 6.2 | 183 |
| 2 | 0 | 0 | 1 | 0 | 1.1 | 4.14 | 7395 | 113.5 | 221 |
| 2 | 0 | 0 | 1 | 0 | 0.8 | 3.6 | 2107 | 139.5 | 188 |
| 2 | 0 | 0 | 1 | 0 | 1 | 3.55 | 1711 | 144.2 | 161 |
| 2 | 0 | 0 | 1 | 0 | 1.9 | 3.92 | 1365 | 144.2 | 122 |
| 2 | 0 | 0 | 1 | 1 | 2.6 | 3.32 | 1110 | 131.8 | 135 |
| 2 | 0 | 0 | 1 | 2 | 3.6 | 2.92 | 996 | 131.8 | 100 |
| 2 | 0 | 0 | 1 | 2 | 4.2 | 2.73 | 860 | 145.7 | 103 |
| 2 | 0 | 0 | 1 | 2 | 3.6 | 2.8 | 779 | 119 | 113 |
| 2 | 0 | 0 | 1 | 2 | 4.6 | 2.67 | 669 | 88 | 100 |
| 3 | 1 | 1 | 0 | 1 | 1.4 | 3.48 | 516 | 96.1 | 151 |
| 3 | 1 | 1 | 1 | 0 | 1.1 | 3.29 | 353 | 69.8 | 160 |
| 3 | 1 | 1 | 0 | 1 | 1.5 | 3.57 | 218 | 57.4 | 107 |

The learning method comprises training the neural network based on the training dataset. The training (or equivalently machine-learning) of the neural network comprises providing entries (e.g., training examples) from the training dataset (obtained in the learning method) as input to the neural network. Each entry of the training dataset comprises one or more time series of values of respective physiological measures of a patient over a past time period (e.g., each time series being a portion of a column corresponding to a patient of the above table). Each of the time series of values is processed through the neural network by multiplying the values with weights, applying activation functions, and combining results across layers. The processing may include a temporal dimension, as the neural network comprises a SNN. In implementations, each entry may comprise baseline data of the patient, and the baseline data may also be processed through the network as explained above. The input layer for the baseline data may be different from the input layer for the time series data. Training may then comprise comparing the output of the neural network against the ground truth of a time occurrence of a clinical event comprised in the training example. The comparison may comprise/compute an error. The comparison may comprise outputting an error based on a loss function. The loss function may quantify the difference between the prediction and the expected outcome. The error (e.g., the outputted error) may then be used to backpropagate through the network, leading to adjustments in the weights to minimize the error. Various optimization solvers, each having their own set of hyperparameters, may be employed during the training process to efficiently converge toward an optimal set of parameters. Hence, as is known *per se* from the field of machine-learning, the training of a neural network (e.g., the neural network of the learning method) comprises tuning the weights, biases, and other parameters of the network so that the processing of an input accurately predicts the occurrence of the clinical event.

The neural network of the learning method is trained for taking as input the same kind of input used during the training, that is, one or more time series of values of respective physiological measures of a patient over a past time period. If the baseline data is used during training, the trained neural network may further take the baseline data as input.

The neural network of the learning method is trained for outputting the same kind of prediction as the ground truth used during the training, that is, a prediction in a (e.g., future) time period of the occurrence of the clinical event. The prediction comprises probabilities (e.g., confidence levels) over a time period of the occurrence of the clinical event. The time period may be a period of time equivalent (e.g., may be the same period of time) to the period of time used in the each of the time series (e.g., a time window, a length of a clinical trial/study) or may be shorter. In implementations, training the neural network may comprise setting a period of time (e.g., in days, or years, e.g., less than 3, or 5 years). The period of time that may correspond to, or be lower than, a time window relative to the time series. In such implementations, the trained neural network may output, for each time point (e.g., each day, or each month) in the period of time, the probability of the occurrence of the clinical event in the time point. The sum of all probabilities over all the time points may be 1 (indicating that

the clinical event is expected to happen in the period of time).

The training of the learning method may comprise minimizing a loss (e.g., a loss function). The loss may penalize, for each training example, a disparity of the prediction outputted by the neural network for the training example and the ground truth for the training example. The loss function may be a distance or any function computing a difference and/or comparison between the output and the ground truth, e.g., based on a mean square error and/or mean absolute error. In implementations, the loss (e.g., loss function) that may be minimized in the learning method may be of the type (i.e., given by the formula):

$$\mathcal{L} = -\frac{1}{n}\sum_{i=1}^{n} \delta^{(i)} \log\left(\hat{f}\left(T^{(i)}\middle|\mathcal{X}_{T^{(i)}}^{(i)}\right)\right) + \left(1-\delta^{(i)}\right)\log\left(\hat{S}^{(i)}\left(T^{(i)}\middle|\mathcal{X}_{T^{(i)}}^{(i)}\right)\right),$$

where $n$ is the number of training examples, $\delta^{(i)}$ indicates the ground truth of occurrence of the medical event (1 if present, 0 otherwise) for the patient $i$ (e.g., the patient associated to the index $i$), $T^{(i)}$ is the time of the event, $\mathcal{X}_t^{(i)}$ represents the data of the training example $i$ up to the time $t$, $\hat{f}(t\,|\mathcal{X}_t^{(i)})$ is the predicted probability of occurrence at time t of the medical event for the training example i knowing all its data up to the time $t$, where

$$f(t\,|\mathcal{X}_t^{(i)}) = \mathbb{P}\left(T^{(i)} = t\,\middle|\mathcal{X}_t^{(i)}, T^{(i)} > t_J^{(i)}\right),$$

with $t_J^{(i)}$ being the time of training example $i$ last known measure, and where $\hat{S}^{(i)}(t\,|\mathcal{X}_t^{(i)})$ is the survival probability of the patient $i$ at time $t$ and is of the type:

$$\hat{S}^{(i)}(t\,|\mathcal{X}_t^{(i)}) = 1 - \hat{F}^{(i)}(t\,|\,\mathcal{X}_t^{(i)}) = 1 - \sum_{s=0}^{t} \hat{f}^{(i)}(s\,|\mathcal{X}_s^{(i)}).$$

These probabilities may constitute the so called "survival function". The above loss function may be well-adapted to the neural network, allowing efficient and improved training of the neural network.

In implementations of the learning method and/or of the method of use, the neural network may comprise (e.g., consists of) several modules (e.g., a spike neural network, additional modules, and/or additional layers to the spike neural network). In such implementations, training the neural network may comprise training each of the several modules, e.g., tuning the weights, biases, and other parameters of each of the several modules. The several modules may be trained all together at the same time during the training of the neural network.

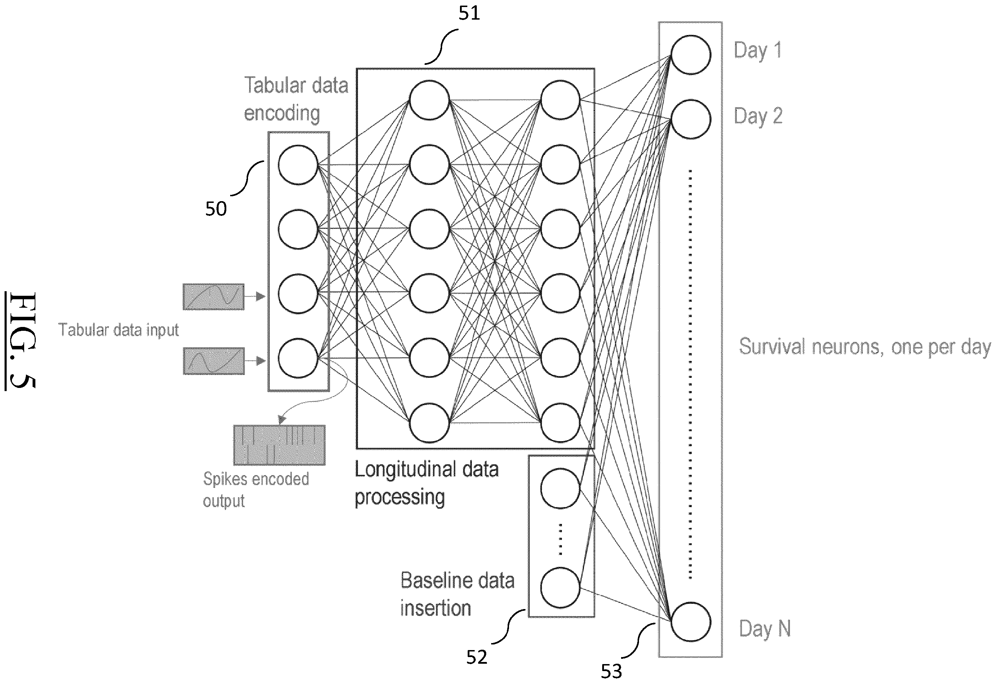

FIG. 5 illustrates an example of an implementation of the neural network trained in the learning method and of the method of use. The neural network of the implementation comprises several modules, including an encoding layer 50, a longitudinal data processing module 51, which may be a SNN, a baseline data insertion module 52, and a last processing module 53.

In implementations of the learning method and/or of the method of use, the neural network may comprise, additionally to the SNN, one or more functions and/or one or more layers that may, e.g., form an additional module. The SNN may be configured to output one or more signals, each comprising a series of spikes. The additional module may be configured to take as input the signals comprising the series of spikes, outputted by the SNN. The additional module may output the prediction, that is, the prediction of probabilities over a time period of the occurrence of the clinical event. The additional module may be trained at the same time as the SNN.

The additional module may comprise a converting function for each spiking neuron of the final layer of the SNN, each converting function taking as input the output of the respective spiking neuron and giving as output a scalar. The converting function may be important, as the output of each of the spiking neurons (that is, the signal comprising a series of spikes) may not be directly processable by further layers of the neural network (e.g., of the additional module). The further layers may expect scalars (e.g., vector of scalars) as input. In implementations, the converting function may be a frequency function. A frequency function may take as input a signal, the signal comprising $N$ occurrences of a spike within a total of $T$ time points, and return the frequency of the spikes, that is, $N/T$. For example, if a signal comprises 20 time points and 15 spikes occurred within these times points, the converting/frequency function may give as output 15/20=0.75. FIG. 4 illustrates an example of a signal having 10 spikes over 20 time points. The converting/frequency function may give as

output in this case 10/20=0.5. Therefore, the frequency functions may be with values in [0,1], however variants of the converting/frequency functions may also be used in other implementations.

The additional module may comprise a fully connected layer, the fully connected layer taking as input the output of the converting functions. Additionally or alternatively, the additional module may comprise a survival neural network (e.g., the survival neural network described in EP4057297A1, which is incorporated herein by reference).

The additional module that may be comprised in the neural network of the method of use and/or learnt in the learning method may apply a SoftMax function to output the prediction. That is, the additional module may comprise a SoftMax function, taking as input the output of a previous layer, and giving as output the prediction. In implementations, the SoftMax function may be applied at the last step in the additional module (e.g., at the last layer). The output of the SoftMax function is a probability distribution. In implementations, such probability distribution may be the output of the neural network, that is, the probability distribution may be the probability over a time period of the occurrence of the clinical event. In implementations, the output of the SoftMax function may indicate, for each time point, the probability that the clinical event occurs at the time point. For example, the additional module may comprise a fully connected layer and/or a survival network and the output of the fully connected layer and/or survival network may be used as input of the SoftMax function.

In an example, the learning method may be used to machine-learn/train a neural network to predict the occurrence of a clinical event (e.g., death) over 300 days. The SNN may have two hidden layer, e.g., having 100 neurons. The additional module may have a final layer, the final layer having 300 neurons, each corresponding to one of the 300 days. The SoftMax function that may be comprised in the additional module may output, for each of the 300 days, the probability of the occurrence of the clinical event on the given day. In implementations, the hyperparameters and the training parameters of the neural network may be the following:

| Hyperparameter | Value |
|---|---|
| Learning rate | 1e-2 |
| Number neurons (first layer) | 5 |
| Number neurons (hidden layer) | 100 |
| Number neurons (last layer) | 300 |
| Number hidden layer | 2 |
| Threshold | 0.1 |
| Tau | 2.0 |
| Dropout | 0.3 |
| Batch size | 128 |

Such an implementation was used in the above-described implementation about the dataset comprising time series of values of physiological measures of patients with metastatic prostate cancer.

Therefore, the additional module may convert the signal outputted by the spiking neurons (that is outputted by the SNN), convert them to scalars, process these scalars, and apply a SoftMax function to create a probability distribution over time. Thus, the additional module may be able to interpret the output of the SNN and convert it into an output understandable by a user.

The additional module may further be configured to take as input the baseline data. The baseline data may be taken as input by the last layer of the additional module, as shown in the module 52 of FIG. 5. In implementations, the fully connected layer and/or the survival neural network that may be comprised in the additional module, may take as input the baseline data, in addition to the output of the SNN (that may have been processed with the above-discussed converting functions). The additional module may be beneficial, as it can combine the output of the SNN, consisting of spikes, and the baseline data, to the actual prediction of the clinical event. This ability to take into account both the longitudinal and baseline data in the same algorithm is an improvement over previous art models. Most of the previous art models do not, and cannot, consider both longitudinal and baseline data simultaneously.

The neural network of the method of use and/or machine-learnt/trained in the learning method may comprise an encoding layer configured for taking as input the one or more time series of values of respective physiological measures of a patient over a past time period and to encode each of the time series into a corresponding signal, i.e., series of spikes. The encoding layer may be important as the time series of values of respective physiological measures comprised in the training dataset and/or provided as input to the trained neural network may comprise real, continuous values, whereas the SNN comprised in the neural network may expect as input signals, that is, may expect as input sequences of spikes (e.g., that may be encoded as binary/trinary signals).

The encoding layer has the considerable advantage of breaking the convention that "spiking neurons always take spike trains as input" and the fact to use spiking neurons to encode data for other spiking neurons.

In implementations of the learning method and/or of the method of use, the encoding layer may consist of a layer that is trained at the same time as the neural network. That is, the training of the neural network comprised in the learning method may additionally comprise training (i.e., tuning the weights and other parameters) the encoding layer. Training the encoding layer at the same time as the rest of the neural network may have the advantage that the weights and parameters are automatically determined by the training and do not have to be manually configured. The process of training may automatically find the optimal weights and parameters to be used in the encoding layer. In the process of training, the weights of the encoding layer may be adjusted to improve encoding.

Alternatively, in other implementations of the learning method and/or of the method of use, the encoding layer may consist of a pre-trained module that is trained independently (and prior to) the neural network. The encoding layer may be trained using a training dataset, the training dataset comprising time series of values of respective physiological measures of a patient and encoded ground truth of spikes. Training the encoding layer independently may have the additional advantage that the same encoded layer may be used for different neural networks.

Alternatively, in other implementations of the learning method and/or of the method of use, the encoding layer may be a deterministic layer, that is, not learnt during the training process.

In implementations of the learning method and/or of the method of use, the encoding layer may comprise delta modulation. Delta modulation may be a method inspired by the way in which the retina senses changes in the field of view and only sends signals when it perceives a change. When no changes are detected, no signals are transmitted. Delta modulation may be based on this premise and may be designed to analyze the variations in a time series to create binary signals.

In implementations of the learning method and/or of the method of use, delta modulation may comprise, given a time series of values of a physiological measure of a patient over a past time period, and for each time point, computing the difference between the values at the time point and at the previous time point. If the difference (or the absolute value of the difference) is greater than a predetermined threshold, then a spike is outputted at the time point. Otherwise, no spike is outputted. In implementations, each spike may be a positive spike or a negative spike. In such implementations, a positive spike is outputted at the time point if the difference is greater than a predetermined positive threshold, and a negative spike is outputted at the time point if the difference is lower than a predetermined negative threshold. In implementations, the predetermined thresholds may be determined in different ways, e.g., may be defined using the mean and standard deviation of differences.

The encoding layer comprising delta modulation that may be comprised in the neural network learned in the method and of the method of use, may be implemented as follows. A time series may be expressed as a sequence of real values $(X_0, ..., X_n)$, where the indices correspond to the time points. That is, each index $t \in [\![0, \ldots, n]\!]$ may correspond to a date (e.g., an hour, a day, or a month). The value $X_t$ is the value of the physiological measure of the patient at the time point t. In implementations, delta modulation may comprise computing the sequence of differences. In formulae, the sequence of differences may be determined as follows:

$$D_0 \coloneqq 0;$$

$$D_t \coloneqq X_t - X_{t-1}, \forall t \in [\![1, \ldots, n]\!].$$

The encoding layer may encode the time series given by the sequence $(X_0, ..., X_n)$ with delta modulation by the signal $S = (S_0, ..., S_n)$ defined as:

$$S_t \coloneqq \begin{cases} 1\ if\ |D_t| \geq T\ and\ t \geq 1, \\ 0\ else. \end{cases}$$

In implementations where spikes may be a positive spikes or negative spikes, the encoding layer may encode the time series given by the sequence $(X_0, ..., X_n)$ with delta modulation with the signals $S = (S_0, ..., S_n)$ defined as:

$$S_t \coloneqq \begin{cases} 1\ if\ D_t \geq T\ and\ t \geq 1, \\ -1\ if\ D_t \leq -T\ and\ t \geq 1, \\ 0\ else. \end{cases}$$

In implementations, the above threshold $T$ may be defined as $T := \overline{D} + \lambda\tilde{D}$, where $\overline{D}$ may be the mean of the

differences $(D_n)_n$, and $\tilde{D}$ may be the standard deviation of the differences $(D_n)_n$. The value $\lambda$ may be a parameter (e.g., a real number) that may be adjusted in the method. The greater the parameter $\lambda$, the fewer spikes may be recorded in the output signal. The parameter $\lambda$ may be, e.g., adjusted with respect to a noise level of the time series.

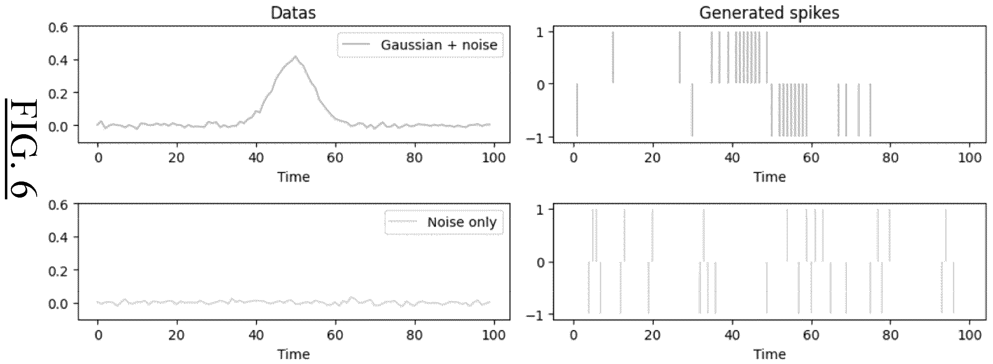

FIG. 6 illustrates the delta modulation method applied to a time series consisting of a noisy Gaussian function (e.g., a sum of a Gaussian function and a white noise function), and to one consisting of a white noise function. As can be seen, delta modulation successfully extracts the variation trend of positive and negative spikes in the case of the Gaussian function, with few spikes due to noise. However, the delta modulation method is less adapted in the case where the time series is a white noise function. In this former case, the time series is globally stationary, but it is encoded with several spikes. The encoding with several spikes is due to a bias in the threshold. Indeed, the threshold is determined using the mean and standard deviation of the sequence of differences.

Alternatively, the encoding layer may be a deterministic layer, that is, not learnt during the training process. The encoding layer may be obtained according to the encoding method, described below in the disclosure. That is, in implementations, the learning method and/or the method of use may comprise an encoder according to the encoding method.

It is further provided a computer-implemented method for encoding a time series of values of a physiological measure of a patient. The method may be referred to as "the encoding method" in the present disclosure.

The encoding method comprises obtaining the time series of values of the physiological measure of the patient. The encoding method further comprises, for each time point of the time series of values, obtaining a slope coefficient of the time series of values at the time point. The obtaining of a slope coefficient is based on (e.g., computed using) the time point (e.g., based on the value of the physiological measure associated to the time point), a predetermined number of previous time points (e.g., based on the values of the physiological measure associated to the predetermined number of previous time points) and a predetermined number of next time points (e.g., based on the values of the physiological measure associated to the predetermined number of next time points). The encoding method further comprises, for each time point of the time series of values, comparing the slope coefficient to a reference slope coefficient. The comparing of the slope coefficient comprises, if the computed slope coefficient is above the reference slope coefficient in absolute value, encoding the time point as a spike. The comparing of the slope coefficient further comprises, if the computed slope is below the reference slope in absolute value, encoding the time point as no spike.

Therefore, the encoding method is a method taking as input a time series of values of a physiological measure of a patient, that may, e.g., be expressed as a vector of real numbers with the indices of the vector corresponding to the time points, and outputting an encoded sequence (e.g., a signal), the encoded sequence comprising a series of spikes. The encoding method may, therefore, be used to implement an encoding layer in a neural network, e.g., the encoding layer discussed above. Thus, the encoding method may further comprise providing the encoded time series of values of the physiological measure of the patient to a neural network (e.g., encoding one or more time series of values of respective physiological measures of a patient and providing each of the encoded time series as input to the neural network). The neural network may comprise a spike neural network, the neural network predicting a time occurrence of a clinical/medical event. The neural network may be the neural network learnt in the learning method and/or of the method of use. In implementations, therefore, the encoding method may be combined with the learning method and/or the method of use (or with steps of either of the methods), so that the encoding method may comprise or be comprised in either of the methods. In such implementations, each of the time series of respective physiological measures of the learning method and/or method of use may be pre-processed with the encoding method. In other implementations, the encoding method may be used to implement an encoding layer in another neural network requiring series of spikes as input, that may, or may not comprise a SNN.

The encoding method is an improved solution for encoding a time series of values of a physiological measure of a patient.

Indeed, contrary to prior art encoding methods, such as the above-described delta modulation method, the encoding method mitigates the effect of unwanted noise in the data. The unwanted noise in the data may be coming from a medical measuring device used to obtain the values of the physiological measure of the patient or may be coming from the process of measuring itself. The mitigation could not have been obtained by previous art methods, e.g., by the delta modulation method. Instead of simply examining the differences in the values of the time series between two consecutive time points, the encoding method comprises obtaining a slope coefficient based on the time point, a predetermined number of previous time points, and a predetermined number of next time points. Thus, the encoding method can analyze the evolution of the time series, through the slope using temporally close data.

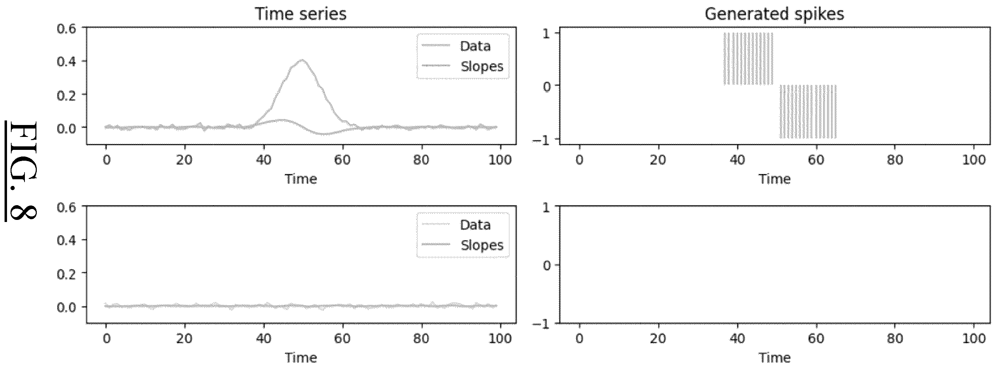

This mitigation is particularly effective as shown in FIG. 8, 9, and 10 discussed herein below. The figures show the efficacy of the encoding method to encode a time series without encoding the unwanted fluctuations of the noise.

The slope coefficients may be the best indicators of the variability of the data, capturing the local trend of the data, e.g., whether the data is substantially changing over time or staying roughly constant. The slope coefficients are calculated based on the time point, a predetermined number of previous time points, and a predetermined number of next time points. Therefore, the slope coefficient encodes the trend of previous and next time points.

Moreover, the flexibility in the choice of the methodology parameters (choice of reference, choice of time window to consider) may allow a better interpretation of our data encoding.

The obtaining of the time series of values of the physiological measure of the patient of the encoding method may comprise retrieving at least part of the time series from (e.g., local or distant) memory or receiving (e.g., from a remote system) at least part of the time series having been thereby acquired. The obtaining may comprise obtaining at least part of the time series directly by measuring a physiological measure of a patient and recording the occurrence of the clinical event for the patient. The obtaining may comprise obtaining at least part of the time series from a clinical trial/study.

The encoding method comprises, for each time point of the time series of values, obtaining a slope coefficient of the time series of values at the time point based on the time point. That is, the encoding method comprises computing a slope coefficient, such as the slope coefficient of a line, at each of the time points of the time series. The slope coefficient is based on the time point, a predetermined number of previous time points, and a predetermined number of next time points.

The predetermined number of previous/next time points may be a predetermined number of immediately previous/next time points, that is, a set of time points immediately preceding/succeeding the time point and of cardinality the predetermined number (e.g., a positive integer).

The predetermined number of previous time points and of next time points may each be a non-negative integer. The predetermined number of previous time points and of next time points may be equal or distinct. In implementations, the predetermined number of previous/next time points may not depend on the base point, e.g., always be the same integer (for all time points, the integer may only depend on "previous" or "next" or be independent of "previous" or "next"). In other implementations, the predetermined number of previous/next time points may depend on the time point. In such implementations, the predetermined number of previous time points may be a number $k_p$ (e.g., $k_p$ may be a non-negative integer), whenever possible (that is whenever there are at least $k_p$ previous time points) and the number of previous time points otherwise. Similarly, in such implementations, the predetermined number of next time points may be a number $k_n$ (e.g., $k_n$ may be a non-negative integer), whenever possible (that is whenever there are at least $k_n$ next time points) and the number of next time points otherwise. In formulae, the time series may be expressed, as already mentioned above, as a sequence $(X_t)_{t=0}^{n}$. Non-negative integers $k_p$ and $k_n$ may be fixed. At the time point $0 \leq t_0 \leq n$, the predetermined number of previous time points may be the number $\min(k_p, t_0)$, and the predetermined number of next time points may be $\min(k_n, n - t_0)$.

Therefore, in implementations, the slope coefficient may be based/compute according to a set of time points comprising the given time points and immediately preceding and succeeding time points. In formulae, the time series may be expressed, as already mentioned above, as a sequence $(X_t)_{t=0}^{n}$, and the slope coefficient based on the time point $t_0$, a predetermined number of previous time points, and a predetermined number of next time points may be based on the elements

$$X_{t_0-\min(k_p,t_0)}, \dots, X_{t_0+\min(k_n,n-t_0)}.$$

In implementations, the slope coefficient may be the slope coefficient of a line. The line may be a best line describing the set of time points comprising (e.g., consisting of) the time point, a predetermined number of previous time points, and a predetermined number of next time points. Therefore, in implementations, the encoding method may comprise determining the best line describing the set of time points $X_{t_0-\min(k_p,t_0)}, \dots, X_{t_0+\min(k_n,n-t_0)}$. The line may be a line in the 2D space in which one dimension is given by the time (i.e., the time points) and the other dimension by the values $X_t$ of the time series.

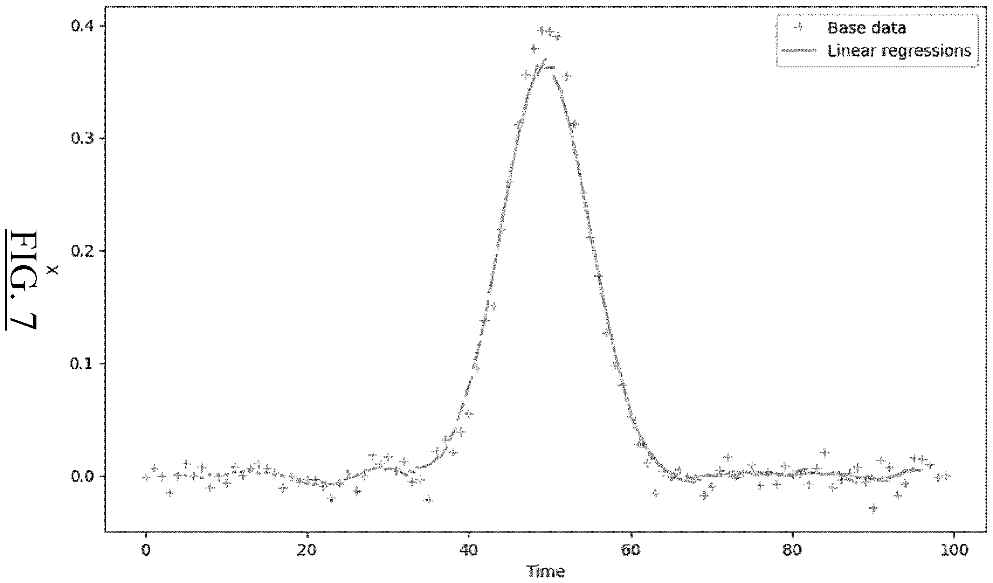

FIG. 7 shows a 2D space comprising points (illustrated with crosses) indicating the values of the time series, each point having as coordinate the time point and the value. FIG. 7 also shows segments at each time point, the segments being representative of the above-discussed lines based at the respective timepoint and indicating the slope coefficient.

The obtaining of a slope coefficient of the time series of values at the time point of the encoding method may comprise computing a linear regression on the values of the time series comprising the time point, the predetermined number of previous time points, and the predetermined number of next time points. The linear regression may be a linear least-squares regression, that is, the linear regression may be a line best approximating the data with respect to the mean square error. Therefore, in implementations, for each time point $t_0$, the encoding method may comprise computing a linear regression (e.g., a linear least-square regression) based on the time points $X_{t_0-\min(k_p,t_0)}, \dots, X_{t_0+\min(k_n,n-t_0)}$ (i.e., on the time points and the associated values of the physiological measure).

Computing a linear regression at each time point may be advantageous as the linear regression may be a best possible line describing the considered data (and the considered metric/error), so the slope of the linear regression may be a best descriptor of the local trends of the time series.

The encoding method further comprises for each time point of the time series of values, comparing the slope coefficient to a reference slope coefficient. Comparing comprises:

o if the computed slope coefficient is above the reference slope coefficient in absolute value, encoding the time point as a spike; and
o if the computed slope is below the reference slope in absolute value, encoding the time point as no spike.

In other words, the encoding method may comprise, for each time point of the time series of values, comparing the variation trend given by the slope coefficient with a reference variation trend. The encoding method may further comprise encoding with a spike if said variation trend is higher than a reference variation.

In formulae, the time series may be expressed, as already mentioned above, as a sequence $(X_t)_{t=0}^{n}$, the slope coefficient at the time point $t$ may be denoted by $m_t$ and the reference slope coefficient by $m_{ref}$. The encoding method may encode the sequence $(X_t)_{t=0}^{n}$ with a signal S = ($S_0$, ..., $S_n$) defined as:

$$S_t := \begin{cases} 1 \; if \; |m_t| \geq m_{ref}, \\ 0 \; else. \end{cases}$$

Each of the spikes of the encoding method may be either a positive spike or a negative spike. The comparing the slope coefficient to a reference coefficient of the encoding method may further comprise:

o if the computed slope coefficient is above a positive reference slope coefficient (e.g., the above-discussed reference slope coefficient in absolute value), encoding the time point as a positive spike; and
o if the computed slope coefficient is below a negative reference slope coefficient (e.g., the opposite of the above-discussed reference slope coefficient in absolute value), encoding the time point as a negative spike.

In other words, the encoding method may further comprise, for each time point of the time series of values, comparing the variation trend given by the slope coefficient with a reference variation. The comparing may consider whether the variation trend is increasing or decreasing. The encoding method may further comprise encoding with a spike if said variation trend is higher than a reference variation.

In formulae, the time series may be expressed, as already mentioned above, as a sequence $(X_t)_{t=0}^{n}$, the slope coefficient at the time point $t$ may be denoted by $m_t$ and the reference slope coefficient by $m_{ref}$. The reference slope coefficient $m_{ref}$ may be assumed positive, similar formulas may be implemented in the other case. The encoding method may encode the sequence $(X_t)_{t=0}^{n}$ with a signal $S$ = ($S_0$, ..., $S_n$) defined as:

$$S_t := \begin{cases} 1 \; if \; m_t \geq m_{ref}, \\ -1 \; if \; m_t \leq -m_{ref}, \\ 0 \; else. \end{cases}$$

The reference slope of the encoding method (e.g., the quantity above referred as $m_{ref}$) may be 1 or a quotient of a maximum amplitude of the values of the time series and a maximum time amplitude. That is the reference slope coefficient may be adapted to the variation of the time series of data. In implementations, the time series of data may not be normalized, and in such implementations, it may be beneficial to consider the maximal variation of the time series for defining the reference slope coefficient.

In formulae, the reference slope coefficient may be $m_{ref}$ = 1. Alternatively, denoting by $(X_t)_{t=t_{min}}^{t_{max}}$ the time series of values of the physiological measure of the patient, the reference slope may be:

$$m_{ref} = \frac{X_{max} - X_{min}}{t_{max} - t_{min}}$$

where $X_{max}$ is the maximum value of the sequence $(X_t)_{t=t_{min}}^{t_{max}}$ and $X_{min}$ is the minimum value of the sequence $(X_t)_{t_{min}}^{t_{max}}$.

FIG. 8 shows the results of an implementation of the encoding method. In FIG. 8, the same time series of values as in FIG. 6 are considered. FIG. 8 shows the encoded spikes as generated by an implementation of the encoding method. The encoded spikes as illustrated in FIG. 8 describe better the variation of the curve than those illustrated in FIG. 6. For example, in FIG. 8, the increasing and decreasing trends of the noisy Gaussian function have been correctly encoded. Moreover, contrary to FIG. 6, that is contrary to prior art methods and specifically the delta modulation method, in FIG. 8 no encoded spikes are encoded due to noise. This is an advantage over the delta modulation method. The delta modulation method may not encode spike data correctly, because of the instability of the data caused by the noise.

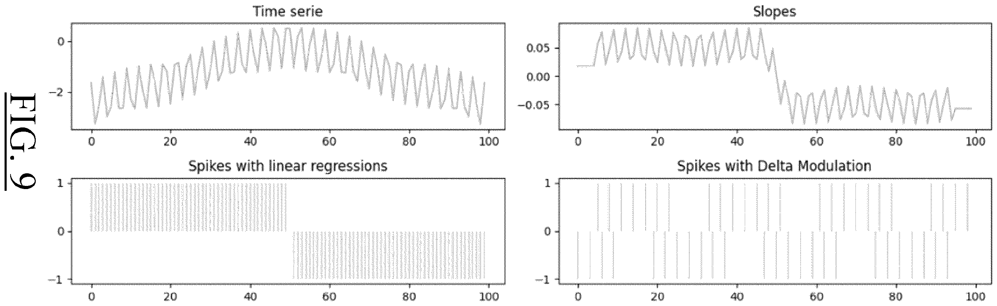

In addition, the encoding method can correctly encode the variation trends of the values of a time series, even if local variations with relatively high amplitudes are frequent. An example is given in reference to FIG. 9.

FIG. 9 shows another example of comparison between the encoding method and prior art methods and specifically the delta modulation method. FIG. 9 shows a time series comprising several oscillations. The variation trend of the time series is well captured in the spikes generated with the encoding method, where positive spikes, indicating an increasing trend, are followed by negative spikes, indicating a negative trend. On the contrary, the delta modulation method generates an output comprising alternating positive and negative spikes and no clear trend can be obtained from the output.

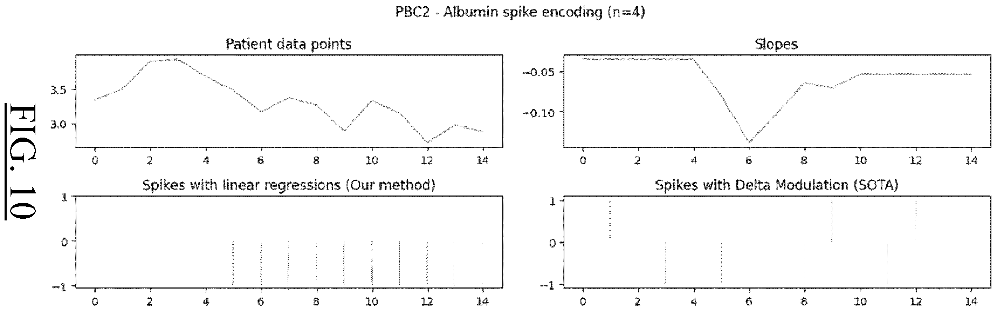

FIG. 10 shows the result of an implementation of the method. In the implementation, public data from patients with primary biliary cirrhosis was used. The data was obtained between January 1974 and May 1984 by the Mayo Clinic (an American university hospital and research federation). The Mayo Clinic conducted a randomized, double-blind study on primary biliary cirrhosis (PBC), comparing the effect of D-penicillamine with a placebo. PBC is a progressive disease of autoimmune origin, and the ensuing inflammatory process ultimately leads to cirrhosis and destruction of the liver's bile. The obtained data comprises longitudinal data (1945 lines of data) of 312 patients. Some patients visited the hospital multiple times. FIG. 10 shows the encoding of the albumin variable, which corresponds to the concentration of albumin in the blood in mg/dl. Albumin is the most abundant plasma protein. In patients with advanced cirrhosis, albumin levels are low due to a reduction in hepatocyte mass caused by the disease itself. In FIG. 10 the encoding method was used taking as the predetermined number of previous time points and the predetermined number of next time points equal to 4. The (positive) reference slope coefficient is 1 and the negative reference slope coefficient is -1. As it can be seen in FIG. 10, the encoding method allows a better description of the overall variation (i.e., the decrease) of the variable on a patient, rather than the prior art delta modulation method. Indeed, the delta modulation method is not able to encode correctly the trend in the variation of the variable.

The methods (e.g., the learning method, the method of use, and the encoding method) are computer-implemented. This means that steps (or substantially all the steps) of each of the methods are executed by at least one computer, or any system alike. Thus, steps of each of the methods are performed by the computer, possibly fully automatically, or, semi-automatically. In examples, the triggering of at least some of the steps of any of the method may be performed through user-computer interaction. The level of user-computer interaction required may depend on the level of automatism foreseen and put in balance with the need to implement user's wishes. In examples, this level may be user-defined and/or pre-defined.

A typical example of computer-implementation of a method is to perform the method with a system adapted for this purpose. The system may comprise a processor coupled to a memory and a graphical user interface (GUI); the memory having recorded thereon a computer program comprising instructions for performing the method. The memory may also store a database. The memory is any hardware adapted for such storage, possibly comprising several physical distinct parts (e.g. one for the program, and possibly one for the database).

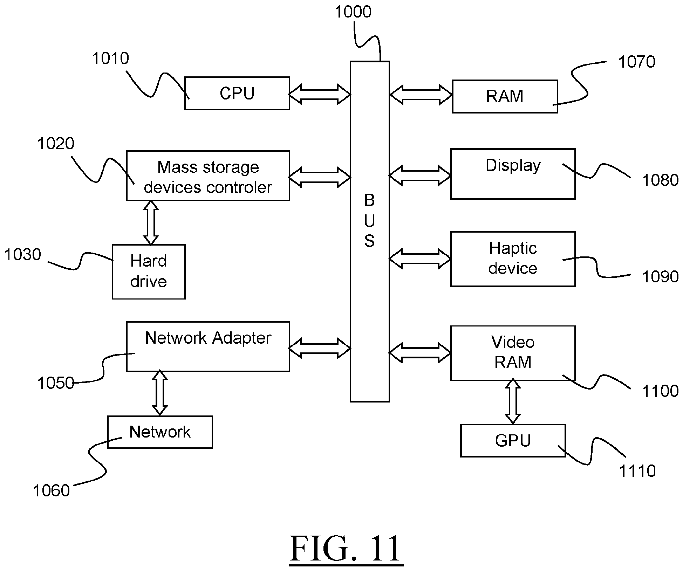

FIG. 11 shows an example of the system, wherein the system is a client computer system, e.g., a workstation of a user.

The client computer of the example comprises a central processing unit (CPU) 1010 connected to an internal communication BUS 1000, a random access memory (RAM) 1070 also connected to the BUS. The client computer is further provided with a graphical processing unit (GPU) 1110 which is associated with a video random access memory 1100 connected to the BUS. Video RAM 1100 is also known in the art as frame buffer. A mass storage device controller 1020 manages accesses to a mass memory device, such as hard drive 1030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 1050 manages accesses to a network 1060. The client computer may also include a haptic device 1090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform any of the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

## Claims

**1.** A computer-implemented method for encoding a time series of values of a physiological measure of a patient, the method comprising:

- obtaining the time series of values of the physiological measure of the patient;
- for each time point of the time series of values, obtaining a slope coefficient of the time series of values at the time point based on the time point, a predetermined number of previous time points, and a predetermined number of next time points; and
- for each time point of the time series of values, comparing the slope coefficient to a reference slope coefficient, the comparing comprising:

  o if the computed slope coefficient is above the reference slope coefficient in absolute value, encoding the time point as a spike; and
  o if the computed slope is below the reference slope in absolute value, encoding the time point as no spike.

**2.** The method of claim 1, wherein the obtaining a slope coefficient of the time series of values at the time point comprises computing a linear regression on the values of the time series comprising the time point, the predetermined number of previous time points, and the predetermined number of next time points.

**3.** The method of claim 1 or 2, wherein the reference slope is 1 or a quotient of a maximum amplitude of the values of the time series and a maximum time amplitude.

**4.** The method of any one of claims 1 to 3, wherein each of the spikes is either a positive spike or a negative spike, and comparing the slope coefficient to a reference coefficient further comprises:

o if the computed slope coefficient is above the reference slope coefficient in absolute value, encoding the time point as a positive spike; and
o if the computed slope coefficient is below the opposite of the reference slope coefficient in absolute value, encoding the time point as a negative spike.

**5.** The method of any one of claims 1 to 4, further comprising providing the encoded time series of values of the physiological measure of the patient to a neural network comprising a Spike Neural Network (SNN) for predicting a time occurrence of a clinical/medical event.

**6.** A computer program comprising instructions which, when executed by a computer system, cause the computer system to perform the method according to any one of claims 1 to 5.

**7.** A computer-readable data storage medium having recorded thereon the computer program of claim 6.

**8.** A computer system comprising a processor coupled to a memory, the memory having recorded thereon the computer

program of claim 6.

Time (t)
Patient 1
Patient 2
Δ difference between visit times
$v_0$
$v_1$
$v_1$
$v_2$
$v_2$
$v_3$
$v_3$
$\Delta_1$
$\Delta_2$
$\Delta_1$
$\Delta_2$
$\Delta_3$


FIG. 1

Death
Relapse
Censorship
Visit
Time (t)
Patient 4
Patient 3
Patient 2
Patient 1
$T_{1,R}$
$T_{2,R}$
$t_3$
$T_{1,D}$
$T_{2,C}$
$\tau$

FIG. 2

Binary Spike
SNN
Spatio-temporal Domain
Synapse
Dendrite
Soma
$u_{th}$
u
S
1 0 1 1
Spikes
$S_0$
1 1 0 1
Pre-spikes
$W_0$
$W_1$
$W_2$


FIG. 3

FIG. 4

Survival neurons, one per day
Day 1
Day 2
Day N
53
51
Longitudinal data processing
Baseline data insertion
52
Tabular data encoding
50
Tabular data input
Spikes encoded output

FIG. 5

Datas
Gaussian + noise
Noise only
Generated spikes
Time
Time
Time
Time
0
20
40
60
80
100
0.6
0.4
0.2
0.0
1
0
−1

FIG. 6

Base data
Linear regressions
0.4
0.3
0.2
0.1
0.0
x
0
20
40
60
80
100
Time

FIG. 7

Time series
Generated spikes
Data
Slopes
Time
0.6
0.4
0.2
0.0
1
0
−1
0
20
40
60
80
100

FIG. 8

Time serie
Slopes
Spikes with linear regressions
Spikes with Delta Modulation
0
−2
0.05
0.00
−0.05
1
0
−1
0
20
40
60
80
100

FIG. 9

PBC2 - Albumin spike encoding (n=4)
Patient data points
Slopes
Spikes with linear regressions (Our method)
Spikes with Delta Modulation (SOTA)

FIG. 10

1000
1010
CPU
1070
RAM
1020
Mass storage
devices controler
B
U
S
Display
1080
1030
Hard
drive
Haptic
device
1090
Network Adapter
Video
RAM
1050
1100
Network
GPU
1110
1060

FIG. 11

# EUROPEAN SEARCH REPORT

Application Number
EP 25 30 5231

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/210552 A1 (KASABOV NIKOLA KIRILOV [NZ] ET AL) 21 July 2016 (2016-07-21) * paragraphs [0007] - [0018], [0201] - [0214], [0248] - [0249] * | 1-8 | INV. A61B5/00 G06N3/049 |
| A | LEHMANN HENDRIK M ET AL: "Analog Spiking Neural Network Based Phase Detector", IEEE TRANSACTIONS ON CIRCUITS AND SYSTEMS I: REGULAR PAPERS, IEEE, US, vol. 69, no. 12, 1 December 2022 (2022-12-01), pages 4837-4846, XP011930092, ISSN: 1549-8328, DOI: 10.1109/TCSI.2022.3204433 [retrieved on 2022-12-01] * the whole document * | 1-8 | |
| A | US 2024/176987 A1 (BANERJEE DIGHANCHAL [IN] ET AL) 30 May 2024 (2024-05-30) * the whole document * | 1-8 | |
| | | | TECHNICAL FIELDS SEARCHED (IPC) |
| | | | A61B G06N |

The present search report has been drawn up for all claims

2

| Place of search | Date of completion of the search | Examiner |
|---|---|---|
| The Hague | 10 June 2025 | Vanderperren, Yves |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 25 30 5231

This annex lists the patent family members relating to the patent documents cited in the above-mentioned European search report. The members are as contained in the European Patent Office EDP file on The European Patent Office is in no way liable for these particulars which are merely given for the purpose of information.

10-06-2025

| Patent document cited in search report | Publication date | Patent family member(s) | Publication date |
|---|---|---|---|
| US 2016210552 A1 | 21-07-2016 | US 2016210552 A1 | 21-07-2016 |
| | | WO 2015030606 A2 | 05-03-2015 |
| US 2024176987 A1 | 30-05-2024 | EP 4386622 A1 | 19-06-2024 |
| | | US 2024176987 A1 | 30-05-2024 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 4057297 A1